# FASCICULE DE BREVET EUROPEEN

(11) **EP 0 462 871 B1**
(45) Date de publication et mention de la délivrance du brevet: **13.09.1995**
(21) Numéro de dépôt: 91401552.4
(22) Date de dépôt: 12.06.1991
(51) Int. Cl.: A61M 3/02

(54) **Dispositif d'hygiène et de traitement**
Hygiene- und Behandlungsvorrichtung
Hygiene and treatment device

(30) Priorité: 19.06.1990 FR 9007624
(43) Date de publication de la demande: 27.12.1991
(73) Titulaire: Ducoin, Jacques, F-21140 Semur en Auxois (FR); Franceschi, Claude, F-92100 Boulogne-Billancourt (FR)
(72) Inventeur: Ducoin, Jacques, F-21140 Semur en Auxois (FR); Franceschi, Claude, F-92100 Boulogne-Billancourt (FR)
(74) Mandataire: Viard, Jean

(56) Documents cités:
- DE-A- 2 343 492
- DE-C- 77 102
- US-A- 3 104 664
- US-A- 3 107 671

## Description

La présente invention a pour objet un dispositif d'hygiène anale ou gynécologique et de traitement permettant un nettoyage simple, hygiénique et non traumatisant sans canulation ni contact avec les organes à traiter.

De telles mesures d'hygiène et de traitement sont recommandées depuis très longtemps et, par exemple dans l'ouvrage intitulé pathologie interne de GRISOLLE paru en 1857 chez Victor Masson. Elles sont destinées au traitement ou à la prévention des hémorroïdes, fissures et irritations anales, des infections gynécologiques et de la constipation basse.

Mais, jusqu'à présent, aucun moyen simple n'a été mis à la disposition du public ou du personnel hospitalier pour effectuer une telle toilette, à part de dispositifs du genre douchette tels que celui illustré, par exemple, dans le document DE-A-2 343 492. La présente invention a pour objet de pallier cet inconvénient.

Selon la présente invention, un dispositif d'hygiène et de traitement est proposé tel que défini dans la revendication 1 Ce dispositif comprend:
- un embout de raccordement à une canalisation d'eau sous pression ;
- une vanne d'arrêt ;
- une chambre à paroi transparente et une canne, coudée à son extrémité opposée à la chambre et présentant une fente longitudinale linéaire au voisinage de cette extrémité.

Ainsi, la canne mise en position, il suffit d'ouvrir la vanne pour que la projection commence. Elle peut être utilisée directement dans la cuvette assurant ainsi des conditions de propreté sans recours à des moyens supplémentaires. La pression de distribution d'eau est ainsi directement utilisée tout en étant modulée par les pertes de charge.

Le dispositif assure par sa forme et son orientation anatomique le nettoyage des parties externes et internes de manière à éviter la rétention même minime des sécrétions et des matières qui mal évacuées constituent des sources d'inconfort et de complications.

D'autres caractéristiques et avantages de l'invention apparaîtront au cours de la description qui va suivre de modes particuliers de réalisation, donné uniquement à titre d'exemples non limitatifs en regard des figures qui représentent :
- La figure 1, une vue d'ensemble du dispositif ;
- La figure 2, une vue éclatée de la chambre à paroi transparente ;
- La figure 3, une vue en coupe de la partie supérieure de la chambre ;
- La figure 4, une vue de détail de la partie supérieure de la canne ;
- La figure 5, une vue de détail de l'extrêmité inférieure de la canne ;
- La figure 6, une variante de réalisation de la commande d'arrivée d'eau.

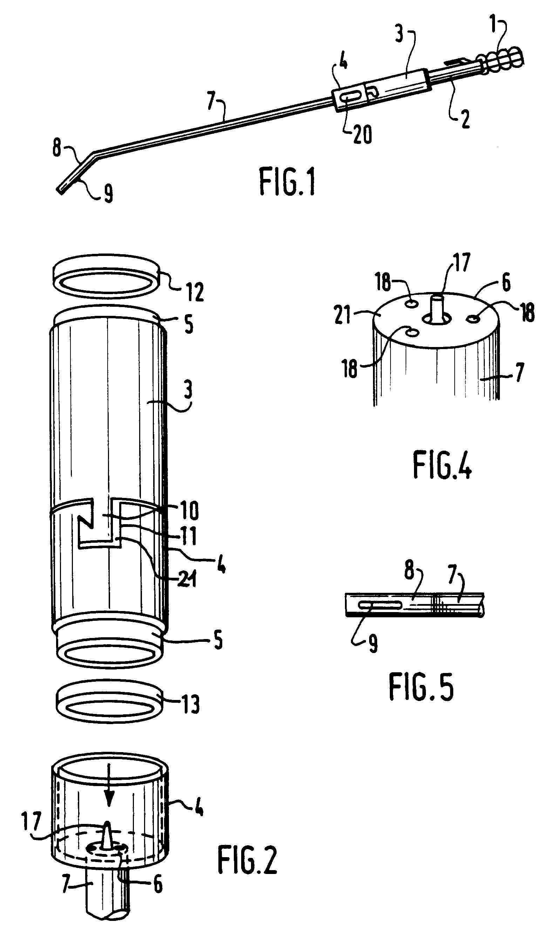

Sur la figure 1, on voit que le dispositif comprend un embout 1 pouvant être raccordé à une, canalisation souple elle-même reliée par l'intermédiaire d'une prise convenable à une canalisation d'eau sous pression. L'embout 1 est monté à la partie supérieure d'un élément 2 pouvant inclure une vanne ou un robinet. L'élément comportant la vanne est relié à un fourreau comprenant deux parties 3 et 4 montées de manière amovible l'une sur l'autre. A la partie inférieure du demi fourreau 4 est fixée une canne 7. Cette canne se termine par une extrémité 8 qui pour des raisons anatomiques est courbée d'un angle d'environ 20° par rapport à l'axe de la canne 7. La canne 7 est constituée par un tube creux obturé à sa partie supérieure c'est-à-dire du côté de la chambre 3, 4 par un disque 21 qui, comme cela apparaît sur la figure 4, est percé de trois trous 18. L'extrémité inférieure 8 de la canne 7 présente par ailleurs une fente longitudinale 9 par laquelle peut s'écouler le liquide, cette fente apparaissant dans la vue de détail de la figure 5. Cette disposition permet d'administrer des douches aux endroits nécessaires en glissant la canne 7 entre les jambes.

Comme cela apparaît sur la figure 2 qui est une vue explosée de la partie médiane de l'appareil le dispositif comprend un fourreau métallique ou en matière plastique, généralement opaque, constitué de parties 3 et 4 et d'un tube transparent 5. Le tube transparent 5 prend appui à ses extrémités inférieure et supérieure sur des rondelles élastiques 12 et 13, par exemple en néoprène, rondelles qui d'une part assurent la fonction de joint d'étanchéité pour le manchon 5 et d'autre part permettent d'obtenir un blocage efficace par coopération entre le tenon 10 et la mortaise 11 constituant une fermeture à baïonnette. Mais selon une caractéristique de l'invention, la patte 10 s'étendant à la partie inférieure du fourreau 3 est de largeur inférieure à celle de la mortaise 11. Il en résulte que lorsque les deux parties 3 et 4 du fourreau sont assemblées, il subsiste une fente 21 formant une lumière verticale. Tout autre forme de fenêtre pourrait être utilisée et par exemple une fenêtre ovale telle que celle qui est représentée sur la figure 1. Le but de cette fenêtre ou lumière est de permettre un contrôle visuel de l'intérieur de la chambre 5. En effet, à l'intérieur de cette chambre peuvent être disposés des médicaments ou antiseptiques de traitement et plus généralement toutes drogues nécessaires au traitement qui sont introduits sous forme de pastilles. Il est utile de pouvoir contrôler l'état de ces produits et notamment leur érosion par le filet d'eau afin de les remplacer lorsque celà devient nécessaire, si, éventuellement, le recours a des médicaments est indiqué.

Afin que ces produits ne viennent obturer les ouvertures 18 représentées à la figure 4 qui ont pour but non seulement de laisser passer l'eau provenant de la canalisation mais également de créer un mouvement tourbillonnaire de cette eau, on a prévu sur le disque 21 un téton central 17 qui empêche toute arrivée face contre face d'une pastille de produits et du disque 21. Les pastilles de produits restent toujours en position inclinée.

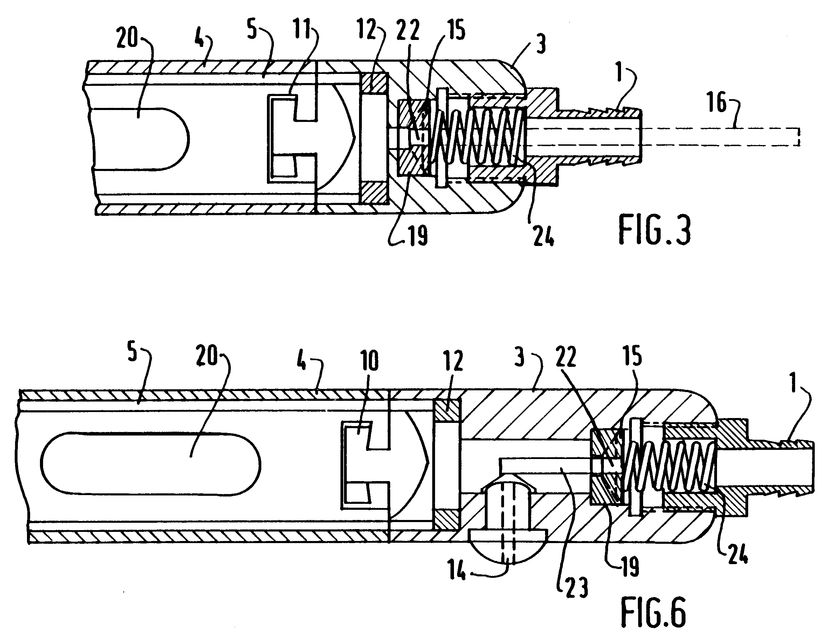

La figure 3 représente en coupe verticale la partie supérieure 3 du fourreau sur lequel est vissé directement, dans ce cas , l'embout 1. L'embout 1 est entouré par une canalisation (non représentée) elle-même reliée au circuit de distribution d'eau. Dans une chambre formée à l'intérieur de la partie 3 du fourreau se trouve, au dessous de l'embout 1, un clapet 15 obturant le conduit 22 de passage de l'eau. Ce clapet 15 est solidaire à sa partie supérieure d'une tige flexible 16. Il est ainsi possible, en tordant légèrement le tuyau d'arrivée d'eau, de déplacer latéralement la tige 16 de manière à soulever le clapet 15. Ainsi, l'ouverture 22 est libérée et l'eau peut s'écouler dans le dispositif.

Une solution alternative au problème de commande de l'arrivée d'eau consiste, comme représenté sur la figure 6 à disposer sur la partie supérieure du fourreau 3 un bouton 14 dont l'extrêmité inférieure est solidaire d'une tige 23. lorsque le bouton 14 est pressé, la tige 23 est déplacée latéralement et le clapet 15 se soulève par rapport au siège 19 ce qui permet le passage de l'eau.

Le fonctionnement de ce dispositif est le suivant:
On vérifie avant l'usage de la canne que les produits devant, éventuellement, se trouver dans la chambre 5 peuvent agir efficacement. Dans le cas contraire, on inclût les produits requis dans la chambre 5 en démontant le fourreau 3, 4 que l'on referme ensuite. La canne étant mise en position, on ouvre alors la vanne 2 éventuellement par action sur le bouton 14 ou en tordant légèrement le tuyau et l'on procède au nettoyage comme indiqué précédemment.

## Revendications

1. Dispositif d'hygiène et de traitement anal sans canulation comprenant une chambre, formée à l'intérieur d'un fourreau (3, 4), mise en communication à l'une de ses extrémités par un raccord (2) avec un embout (1) d'une canalisation d'eau sous pression, et des moyens de fermeture et d'ouverture de l'eau, caractérisé en ce que la seconde extrémité de la chambre est reliée à une canne longiligne (7) dont l'extrémité est incurvée, présentant une fente longitudinale (9) de sortie unique de l'eau, du côté intérieur à la partie incurvée, au voisinage de ladite extrémité.

2. Dispositif selon la revendication 1 caractérisé en ce que les deux parties (3, 4) de la chambre sont réunies par un montage à baïonnette (10, 11).

3. Dispositif selon la revendication 2 caractérisé en ce que la largeur de l'élément male (10) de la fermeture à baïonnette est inférieure à la largeur (11) de l'élémemt femelle de manière à créer une lumière longitudinale (21).

4. Dispositif selon l'une quelconque des revendications précédentes, caractérisé en ce qu'un manchon transparent (5) est monté à l'intérieur du fourreau (3,4), une fenêtre (20) permettant de contrôler le contenu de la chambre.

5. Dispositif selon l'une quelconque des revendications précédentes, caractérisé en ce que la partie supérieure de la canne (7) est obturée par un disque (21) percé de trous (18), un téton (17) faisant saillie dans la partie centrale du disque (21).

## Claims

1. A device for anal hygiene and treatment without canulation, the device comprising a closed chamber inside a sheath (3, 4), that is put into communication at one of its ends via a coupling (2) with an endpiece (1) of a pipe for water under pressure, and means for turning the water on and off, the device being characterized in that the second end of the chamber is connected to an elongate stick (7) whose end is curved, having a single longitudinal outlet slot (9) for water on the inside of the curved portion, in the vicinity of said end.

2. A device according to claim 1, characterized in that the two portions (3, 4) of the chamber are united by a bayonet fastening (10, 11).

3. A device according to claim 2, characterized in that the width of the male element (10) of the bayonet fastening is less than the width (11) of the female element so as to establish a longitudinal opening (21).

4. A device according to any preceding claim, characterized in that a transparent sleeve (5) is mounted inside the sheath (3, 4), a window (20) enabling the contents of the chamber to be inspected.

5. A device according to any preceding claim, characterized in that the top portion of the stick (7) is closed by a disk (21) pierced by holes (18), a stud (17) projecting from the central portion of the disk (21).

## Patentansprüche

1. Vorrichtung zur Hygiene und Analbehandlung ohne Kanülisierung, mit einer im Inneren einer Hülse gebildeten Kammer (3, 4), die an einem ihrer Enden durch ein Verbindungsstück (2) mit einem Ansatz (1) mit einer Druckwasserleitung in Verbindung steht und mit Schließ- und Öffnungsmitteln für das Wasser, dadurch gekennzeichnet, daß das zweite Ende der Kammer mit einem länglichen Rohr (7) in Verbindung steht, dessen Ende abgebogen ist und einen Längsschlitz (9) ausschließlich zum Austritt von Wasser an der Innenseite des abgebogenen Teiles und in der Nähe des Endes aufweist.

2. Vorrichtung nach Anspruch 1, dadurch gekennzeichnet, daß die beiden Teile (3, 4) der Kammer durch einen Bajonettverschluß (11, 12) miteinander verbunden sind.

3. Vorrichtung nach Anspruch 2, dadurch gekennzeichnet, daß die Größe des männlichen Elementes (10) des Bajonettverschlusses kleiner als die Breite (11) des weiblichen Elementes ist, so daß ein Längsspiel (21) erzeugt wird.

4. Vorrichtung nach einem der vorhergehenden Ansprüche, dadurch gekennzeichnet, daß eine durchsichtige Buchse (5) im Inneren der Hülse (3, 4) angeordnet ist und ein Fenster (20) den Inhalt der Kammer zu überwachen erlaubt.

5. Vorrichtung nach einem der vorhergehenden Ansprüche, dadurch gekennzeichnet, daß der obere Teil des Rohres (7) von einer Scheibe (21) abgedeckt ist, die mit Löchern (18) durchdrungen ist und daß ein Ansatz (17) über den zentralen Teil der Scheibe (21) hinausragt.
